# EUROPEAN PATENT APPLICATION

(11) **EP 4 049 640 A1**
(43) Date of publication of application: **31.08.2022**
(21) Application number: 22163448.8
(22) Date of filing: 31.01.2014
(51) Int. Cl.: A61F 13/534, A61F 13/539

(54) **NONWOVEN CARRIER FOR ABSORBENT ARTICLE**

(62) Divisional of application: 18160503.1
(71) Applicant: Ontex BV, 9255 Buggenhout (BE)
(72) Inventor: WEBER, Ainas, 53474 Bad Neuenahr-Ahrweiler (DE); DE POORTER, Annick, 9320 Aalst-Erembodegem (BE); MAILINGER, Christel, 65604 Elz (DE)
(74) Representative: Larangé, Françoise

(57) **Abstract**

The present invention relates to a nonwoven carrier for immobilizing superabsorbent polymer particles in an absorbent article, to an absorbent article such as a diaper made of such carrier, to methods for manufacturing said carrier and said absorbent article, as well as to the use of a nonwoven carrier according to the invention for immobilizing superabsorbent polymer particles in an absorbent article, particularly a diaper.

## Description

### TECHNICAL FIELD

The present invention relates to a nonwoven carrier for immobilizing superabsorbent polymer particles in an absorbent article, to an absorbent article such as a diaper made of such carrier, to methods for manufacturing said carrier and said absorbent article, as well as to the use of a nonwoven carrier according to the invention for immobilizing superabsorbent polymer particles in an absorbent article, particularly a diaper. The present invention is of particular importance to the field of hygiene products, in particular diapers.

### BACKGROUND

The use of superabsorbent polymer material in disposable diapers is well known. The use of superabsorbent polymer material facilitates disposable diapers having a thinner absorbent core versus the use of absorbent materials such as fluff pulp, especially while the disposable diaper is in the dry state.

Today, most disposable diapers which are commercially available have absorbent cores containing a mixture of cellulose fibers and superabsorbent polymer particles. The cellulose fibers comprised by the absorbent core partly hold the particles in place. The particles are not completely immobilized and may still have some degree of freedom to move within the interstices of the cellulose fibers.

Prior absorbent structures have generally comprised relatively low amounts (e.g., less than about 50 % by weight) of these superabsorbent polymer particles. There are several reasons for this. The superabsorbent polymer particles employed in prior absorbent structures have generally not had an absorption rate that would allow them to quickly absorb body fluids, especially in "gush" situations. This has necessitated the inclusion of fibers, typically wood pulp fibers, to serve as temporary reservoirs to hold the discharged fluids until absorbed by the superabsorbent polymer particles. More importantly, many of the known superabsorbent polymer particles exhibited gel blocking. "Gel blocking" occurs when superabsorbent polymer particles are wetted and the particles swell so as to inhibit fluid transmission to other regions of the absorbent structure. Wetting of these other regions of the absorbent member therefore takes place via a very slow diffusion process. In practical terms, this means acquisition of fluids by the absorbent structure is much slower than the rate at which fluids are discharged, especially in gush situations. Leakage from the absorbent article can take place well before the particles of SAP in the absorbent member are even close to being fully saturated or before the fluid can diffuse or wick past the "blocking" particles into the rest of the absorbent member. Gel blocking can be a particularly acute problem if the superabsorbent polymer particles do not have adequate gel strength and deform or spread under stress once the particles swell with absorbed fluid.

These gel blocking phenomena have typically necessitated the use of a fibrous matrix in which are dispersed the superabsorbent polymer particles. This fibrous matrix keeps the superabsorbent polymer particles separated from one another. This fibrous matrix also provides a capillary structure that allows fluid to reach the superabsorbent polymer particles located in regions remote from the initial fluid discharge point. However, dispersing the superabsorbent polymer particles in a fibrous matrix at relatively low concentrations in order to minimize or avoid gel blocking can lower the overall fluid storage capacity of thinner absorbent structures. Usage of lower concentrations of these superabsorbent polymer particles limits somewhat the real advantage of these materials, namely their ability to absorb and retain large quantities of body fluids per given volume. Another reason why extremely high concentrations of superabsorbent polymer particles were not possible resides in the physical integrity disadvantage of structures made of particulate material. Creating a fibrous matrix therefore also had the advantage of providing a fiber re-enforced structure, similar to those used in many other technical situations where structural re-enforcement is provided by fibrous elements, such as in fiberglass.

Using higher amounts of superabsorbent polymer particles is desirable as it enables thinner absorbent cores. However, in absorbent cores having high amounts of superabsorbent polymer particles and litter or no cellulose fibers, the superabsorbent polymer particles can no longer be held in place, as the ratio of particles to fibers is too high. In absorbent cores with very high amounts of superabsorbent polymer particles, such as more than 80%, the particles are generally immobilized by the use of an adhesive, such as a hot melt adhesive, applied as a fine, fibrous network within the absorbent core. The application of adhesive, in particular hot melt adhesive, has a number of disadvantages, including (i) the potential to inhibit fluid distribution, (ii) process issues due to dirt and blocking of nozzles during production, and (iii) high costs. In addition, the polymer material is sandwiched between carrier substrates. The carrier substrates are typically nonwoven webs.

It has been found that the dense structure of the superabsorbent polymer particles causes winding or tearing issues even at low line speed in a diaper machine. Therefore use of nonwoven webs filled with superabsorbent polymer particles are preferably not processed on rolls.

Furthermore, absorbent cores with a high amount of superabsorbent particles and a low amount of fluff, and fluffless absorbent cores generally suffer from a lack of softness.

There is thus a need for disposable diapers with absorbent cores with superabsorbent polymer particles, to be more easily processable. Moreover there is still a need for alternative fixation of the superabsorbent polymer particles. There is also a need for an absorbent core with a high amount of superabsorbent particles, which retains a degree of softness.

The present invention aims to resolve at least some of the problems mentioned above.

The invention thereto aims to provide an improved absorbent core and process for making such a core.

### SUMMARY OF THE INVENTION

The present invention concerns an absorbent article, preferably a disposable absorbent article, such as a diaper. In a first aspect, the present invention specifically concerns a nonwoven carrier (1) for immobilizing superabsorbent polymer particles (28) in an absorbent article, comprising a top layer (7) consisting of staple and/or spunbond fibers (19) penetrable by the particles and a nonwoven bottom layer (8) bonded, such as thermally bonded, bonded by melting by heat or pressure or ultrasonics, bonded by needlepunching, chemically bonded and/or bonded by adhesives, preferably mechanically bonded, more preferably hydroentangled to the top layer; wherein the bottom layer is porous with a pore size smaller than the particles.

A nonwoven carrier according to the invention provides an at least two-layered structure with very different properties. The top layer is porous, soft and bulky. In contrast, the bottom layer has much smaller pores, but considerably higher mechanical strength.

In a second aspect, the present invention provides an absorbent article comprising a nonwoven carrier according to an embodiment of the invention immobilizing superabsorbent polymer particles.

The superabsorbent polymer particles can be trapped in between the fibers of the upper layer, thereby preventing them from moving in the longitudinal and transversal direction (x-y plane) of the absorbent article. The particles are kept from falling through the material, i.e. kept from moving in a cross-sectional direction (z-direction).

In a third aspect, a process for manufacturing a nonwoven carrier according to an embodiment of the invention, comprising the steps of: providing a nonwoven bottom layer and a top layer comprising staple fibers, and mechanically bonding the staple fibers to the nonwoven bottom layer thereby providing the nonwoven carrier in which the superabsorbent polymer particles are retained.

The mechanical bonding of the staple and/or spunbond fibers to the bottom layer provides for an essentially permanent fixation of staple fibers to a nonwoven carrier. A robust structure for trapping and holding superabsorbent polymer particles is provided.

In a further aspect, the invention also provides a process for manufacturing an absorbent article according to an embodiment of the invention, comprising the steps of: providing a nonwoven carrier and providing the nonwoven carrier with superabsorbent polymer particles.

The process provides a way to distribute superabsorbent polymer particles over the nonwoven carrier structure of the invention. Rather than only lying on the surface of the top layer, particles will also be trapped inside the fibers. Their distribution is limited by the bottom layer. The pore size of the bottom layer is smaller than the particle size of the superabsorbent polymer particles, preventing the particles from falling through the material.

In a final aspect, the invention provides use of a nonwoven carrier according to an embodiment of the invention for immobilizing superabsorbent polymer particles in a diaper.

The nonwoven carrier provided by the invention can be particularly advantageous for use in an absorbent article such as a diaper.

Preferred embodiments are as specified in the dependent claims.

### BRIEF DESCRIPTION OF THE DRAWINGS

The invention will be further described in detail with reference to the exemplary embodiments represented by the accompanying figures, in which
**FIG. 1** shows a schematic cross-sectional view of an absorbent article according to the present invention.
**FIG. 2** shows a schematic cross-sectional view of a core wrap including a nonwoven carrier belonging to the absorbent article from **FIG. 1** according to the present invention.
**FIGS. 3a****-g** shows a technical drawing of different patterns of clusters of superabsorbent polymer particles.
**FIG. 4** shows a plan view of an embodiment of an absorbent article according to the present invention.
**FIG. 5** shows a plan view of an embodiment of another type of absorbent article according to the present invention.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refer to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specify the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like.

The absorbent article of the present invention preferably comprises a liquid permeable top sheet, a liquid impermeable back sheet, and an absorbent medium disposed between the top sheet and the back sheet. The absorbent medium may comprise a nonwoven carrier according to the present invention. The absorbent article may also include one or more features such as, but not limited to, ears or side panels, leg cuffs, fastener components and/or a belt. The top sheet, back sheet and the absorbent medium could be made from any suitable material known to the person skilled in the art.

"Absorbent medium" is the absorbent structure disposed between the top sheet and the back sheet of the absorbent article in at least the crotch region thereof. The absorbent material can be of any conventional kind. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material. Superabsorbent polymers are water-swellable, water-insoluble organic or inorganic materials capable of absorbing at least about 20 times their own weight of an aqueous solution containing 0.9 weight percent of sodium chloride. Organic materials suitable for use as superabsorbent materials can include natural materials such as polysaccharides, polypeptides and the like, as well as synthetic materials such as synthetic hydrogel polymers. Such hydrogel polymers include, for example, alkali metal salts of polyacrylic acids, polyacrylamides, polyvinyl alcohol, polyacrylates, polyvinyl pyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly cross-linked to render the material substantially water insoluble. Preferred superabsorbent materials are further surface cross-linked so that the outer surface or shell of the superabsorbent particle, fibre, flake, sphere, etc. possesses a higher crosslink density than the inner portion of the superabsorbent. The superabsorbent materials may be in any form which is suitable for use in absorbent composites including particles, fibres, flakes, spheres, and the like.

Preferably, the absorbent medium comprises a nonwoven carrier according to the present invention.

"Diaper" refers to an absorbent article generally worn by infants and incontinent persons about the lower torso.

"Disposable" is used herein to describe articles that are generally not intended to be laundered or otherwise restored or reused (i. e. they are intended to be discarded after a single use and, preferably, to be recycled, composted or otherwise disposed of in an environmentally compatible manner).

"Nonwoven" refers to a manufactured sheet, web, or batt of directionally or randomly oriented fibres bonded by friction and/or cohesion and/or adhesion, excluding paper and products that are woven, knitted, tufted, stitchbonded incorporating binding yarns or filaments, or felted by wet-milling, whether or not additionally needled. The fibres may be of natural or man-made origin. They may also be staple or continuous filaments or be formed in situ.

The term "top sheet" refers to a liquid permeable material sheet forming the inner cover of the absorbent article and which in use is placed in direct contact with the skin of the wearer. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. Further examples of top sheet materials are porous foams, apertured plastic films, laminates of nonwoven materials and apertured plastic films etc. The materials suited as top sheet materials should be soft and nonirritating to the skin and be readily penetrated by body fluid, e.g. urine or menstrual fluid. The inner coversheet may further be different in different parts of the absorbent article. The "back sheet" refers to a material forming the outer cover of the absorbent article. The back sheet may be the same or different in different parts of the absorbent article. At least in the area of the absorbent medium, the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The "staple fibers" refer to commercially available fibers that are filaments having diameters ranging from less than about 0.001 mm to more than about 0.2 mm; they come in several different forms such as short fibers ranging from about 10 to 50 mm in length and long fibers with a length higher than 50 mm, preferably up to 100 mm.

The "spunbond fibers" refer to generally continuous fibers which can be produced by the extrusion of molten polymer from either a large spinneret having several thousand holes per meter of width or with banks of smaller spinnerets, for example, containing as few as 40 holes. After exiting the spinneret, the molten fibers are quenched by a cross-flow air quench system, then pulled away from the spinneret and attenuated by high speed air. The average diameter of spunbond fibers is typically in the range of from 15-60 µm or higher.

The staple and/or spunbond fibers for use in the invention can be prepared from light and/or heavy fibers, preferably polypropylene and/or polyester fibers. The light fibers have a dtex below 3, preferably in the range of 1 to 3 whereas the heavy fibers have a dtex of at least 3 and preferably lighter than 45 dtex. In case a blend of light and heavy fibers is used, the heavy fibres preferably have a dtex value which is at least two times, but no greater than 15 times that of the light fiber.

The "hydroentanglement process" refers to the manufacturing of nonwoven webs. The process involves directing a series of water jets towards a fibrous web which is supported on a moving porous belt. The water jets pass downwards through the mass of fibres and on making contact with the surface of the belt, the jets rebound, and break up: the energy released causes entanglement of the mass of fibres.

"Superabsorbent polymer particles" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc..., the particle size preferably ranges between 100 to 1500 *µ*m or beyond, preferably between 100 and 800 *µ*m, preferably between 300 to 600 *µ*m, more preferably between 400 to 500 *µ*m.

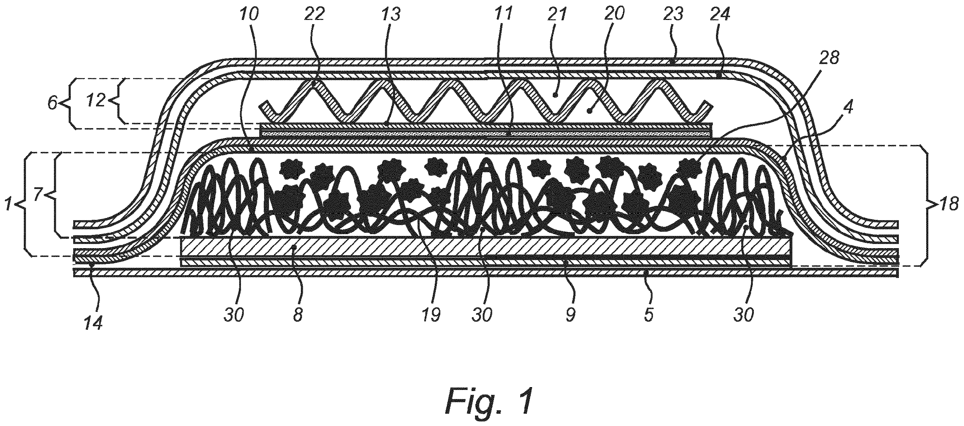

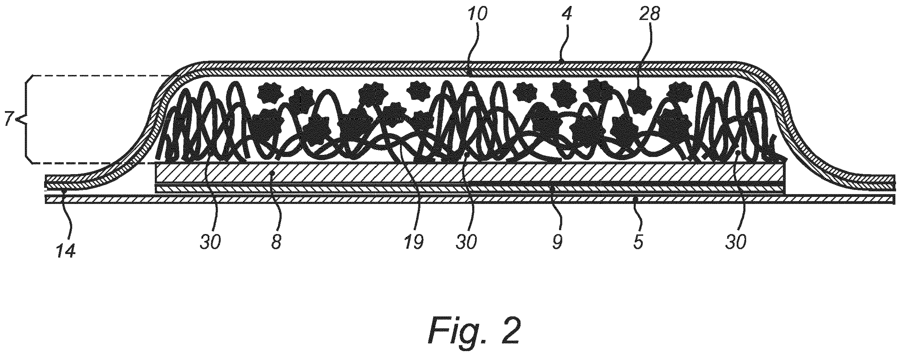

**Fig. 1** and **2** show two embodiments of an absorbent article according to the present invention. **Fig. 1** is a general schematic cross-sectional view of the absorbent article whereas **Fig. 2** is a detailed part of the schematic cross-sectional view of **Fig. 1** showing the lower part of the absorbent article.

The inventors have found a way to provide an improved absorbent core and process for making such a core.

In particular, the present invention provides in a first aspect an absorbent core (18) including a nonwoven carrier (1) for immobilizing superabsorbent polymer particles (28) in an absorbent article, comprising a top layer (7) comprising staple and/or spunbond fibers (19) penetrable by the superabsorbent polymer particles and a nonwoven bottom layer (8), which is preferably mechanically bonded to the top layer **(****Fig. 1**). The absorbent core (18) is wrapped between two layers of nonwoven, the so-called core wrap. The lower core wrap (5) is attached to the bottom layer (8) of the nonwoven carrier (1) by thermoplastic adhesive (9). The upper core wrap (4) is attached to the top surface of the nonwoven carrier (1) by thermoplastic adhesive (10). The superabsorbent polymer particles (28) can be partly in contact with the adhesive coating (10) on the upper core wrap (4).

In a preferred embodiment, the widths of the core wrap nonwovens exceed the width of the nonwoven carrier (1), so that the core wrap nonwovens can be bonded with each other along the side edges by thermoplastic adhesive (14).

In a preferred embodiment, a wrap nonwoven is at least partially and preferably completely folded around the core and sealed on top or bottom.

In a preferred embodiment, a cup-shape configuration is provided whereby the side edges of the lower core wrap are folded on top of the core.

In an alternative embodiment, no core wrap is provided.

On top of the absorbent core (18), an acquisition and distribution layer (ADL) (6) is placed which is attached by thermoplastic adhesive (11). Preferably, the ADL (6) is based upon Nonwovens Innovation & Research Institute's proprietary Hydrospace technology as described in EP 1644564A1. Note however, that any type of adhesive application knwion in the art could be used to attach the ADL to other components or layers of an absorbent article. In particular, adhesion can be obtained by gluing, such by contact coating with full coverage or partial coverage, e.g. in stripes, or by spray coating, e.g. random spray coating or along a pattern, continuous or discontinuous, e.g. by non-continuous lines of spiral spray. Adhesion can also be obtained by alternative bonding techniques, such as thermal bonding, thermo-mechanical bonding, mechanical bonding and/or ultrasonic bonding.

The absorbent core (18) and the ADL (6) are covered by a topsheet nonwoven (23) which is attached to the components underneath with a layer of thermoplastic adhesive (24), by thermobonding and/or by ultrasonic bonding.

The nonwoven carrier (1) according to the invention provides an at least two-layered structure with very different properties **(****Fig. 2****).** The top layer (7) is porous, soft and bulky. In contrast, the bottom layer (8) has much smaller pores than the superabsorbent polymer particles (28) and acts as a barrier. The pore size of the bottom layer (8) is smaller than the particle size of the superabsorbent polymer particles, preventing the particles from falling through the material. The particle size of the particles is preferably between 100 to 800 µm. In addition, the bottom layer (8) has a higher mechanical strength, adding robustness to the nonwoven carrier (1). As a result, the superabsorbent polymer particles (28) are trapped in between the staple fibers (19) of the top layer (7) that kept them from moving in the longitudinal and transversal direction (x-y plane) of the absorbent article. Moreover, the superabsorbent polymer particles (28) are kept from falling through the material, i.e. kept from moving in a cross-sectional direction (z-direction).

In a preferred embodiment, the bottom layer is a spunmelt fabric, preferably a spunbond meltblown spunbond (SMS) fabric. The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated in the SMS fabric, for example spunbond-meltblown-meltblown-spunbond (SMMS), etc.

This multi-layered composite sheet product contribute strength to the overall composite due to the exterior layers of the SMS or SMMS nonwoven fabric, while the middle or core layer comprises a meltblown web that provides barrier properties. The spunmelt bottom layer (8) acts thus effectively as a barrier for the superabsorbent polymer particles (28), but also adds robust mechanical properties to the nonwoven carrier (1). As a result, the nonwoven carrier (1) can be converted as roll good on a diaper machine without experiencing winding or tearing issues even at high line speed, e.g. 7 m/s. Owing to the high resilience of the fibers (19) employed, as well as to the bonding with the bottom layer (8), the top layer (7) is likely to recover its lofty and porous state even after being spooled and compressed on a roll. As a result, the superabsorbent polymer particles (28) can penetrate into and become entangled between the fibers (19), rather than only lying on the surface of the top layer (7). The bottom layer (8) acts as a barrier so that the superabsorbent polymer particles (28), to a large extent, get trapped inside the nonwoven carrier (1).

SMS or SMMS nonwovens are typically composed of a thermoplastic polymer, such as polypropylene, which causes the nonwoven to be naturally hydrophobic. As a result, the SMS or SMMS nonwovens are treated with a topical hydrophilic treatment to increase absorption, such as a surfactant treatment. In a preferred embodiment, the grammage of the top layer (7) is 30 gsm.

In a preferred embodiment, the grammage of the top layer (7) is at least 1 gsm, preferably at least 5 gsm, more preferably at least 10 gsm, more preferably at least 15 gsm, even more preferably at least 20 gsm, and/or preferably at most 200 gsm, more preferably at most 100 gsm, more preferably at most 50 gsm, even more preferably at most 40 gsm, e.g. 20, 25, 30, 35, 40 gsm, most preferably about 30 gsm.

In a preferred embodiment, the grammage of the bottom layer (8) is 8 gsm.

In a preferred embodiment, the grammage of the bottom layer (8) is at least 0,5 gsm, preferably at least 1 gsm, more preferably at least 2 gsm, more preferably at least 3 gsm, more preferably at least 4 gsm, even more preferably at least 5 gsm, and/or preferably at most 50 gsm, more preferably at most 40 gsm, even more preferably at most 30 gsm, even more preferably at most 20 gsm, even more preferably at most 12 gsm, e.g. 5, 6, 7, 8, 9, 10, 11, 12 gsm, most preferably about 8 gsm.

In a preferred embodiment, the staple fibres comprise a blend of two types of fibers, preferably polyester fibers, polyethylene fibers and/or polypropylene fibers. Preferably, a first type of fibres is short, more preferably shorter than 50 mm, yet more preferably shorter than 47 mm, still more preferably shorter than 43 mm, even more preferably shorter than 40 mm, most preferably about 38 mm, and/or said first type of fibres is fine, more preferably thinner than 3 dtex, still more preferably thinner than 2.5 dtex, yet more preferably thinner than 2 dtex, yet even more preferably thinner than 1.5 dtex, most preferably about 1.3 dtex. Also preferable, a second type of fibres of said two types is long, more preferably longer than 50 mm, yet more preferably longer than 55 mm, still more preferably longer than 60 mm, even more preferably longer than 65 mm, most preferably about 68 mm, and/or said second type of fibres is coarse, more preferably thicker than 3 dtex, still more preferably thicker than 4 dtex, yet more preferably thicker than 5 dtex, yet even more preferably thicker than 6 dtex, most preferably about 6.7 dtex. In a particular preferred embodiment, said first type of fibres is short and fine, most preferably about 38 mm and 1.3 dtex and second type of fibres is long and coarse, most preferably about 68 mm and 6.7 dtex.

In a more preferred embodiment, the blend comprises between 10% and 50%, more preferably between 20% and 40%, yet more preferably between 25% and 35%, most preferably about 30% of the first type of fibres and/or 50% to 90%, more preferably between 60% and 80%, yet more preferably between 65% and 75%, most preferably about 70% of the second type of fibres.

Preferably, the staple fibers comprise a substantially round cross section, a substantially trilobal cross section and/or a substantially quadrilobal cross section.

In a preferred embodiment, the nonwoven carrier is rolled up and thereby typically compressed. The nonwoven carrier devoid of superabsorbent polymer particles at this stage can be better transported. The composition of the top layer (7) provides for a good recovery after compression. In a preferred embodiment, the top layer (7) comprises a blend of two different types of staple fibers: one type is short and fine, whereas the other type is long and coarse. In a preferred embodiment, the top layer (7) is mechanically bonded to the bottom layer (8) by hydroentanglement. The short and fine fibers included in the top layer (7) are particularly advantageous to achieve good bonding strengths between the top layer (7) and the bottom layer (8) in the hydroentanglement process. Preferably, the short and fine fibers have a crimp frequency of 12/cm, and the long and coarse fibers have a crimp frequency of 3.5/cm. The recovery after compression of highloft nonwoven fabrics can be determined with the EDANA standard method WSP 120.R4 (12). In a preferred embodiment, the recovery of the nonwoven carrier (1) according to this method is greater than 90%.

In a preferred embodiment, the nonwoven carrier is comprising superabsorbent polymer particles which are at least partially immobilized by the staple fibers and retained by the bottom layer. The superabsorbent polymer particles are trapped inside the fibers. That limits their movement within the absorbent article, both in the longitudinal and the transversal directions (x-y plane) as well as up or downward (z-direction). The bottom layer acts as an additional barrier. The average size of the pores of the bottom layer is smaller than the average particle size of the superabsorbent polymer particles, so that even the fine fraction of the particles is prevented from falling through the material. The sizes of superabsorbent polymer particles depend on the exact application, but for absorbent articles such as diaper, the main fraction is ranging between 100 to 800 *µ*m, preferably between 300 to 600 *µ*m, more preferably between 400 and 500 *µ*m.Therefore, in a preferred embodiment, the bottom layer has a pore size smaller than 300 *µ*m, more preferably smaller than 100 µm, yet more preferably smaller than 50 µm, still more preferably smaller than 25 µm, even more preferably smaller than 10 µm, thereby retaining the superabsorbent polymer particles. In a more preferred embodiment, said SAPs are distributed in a pattern, preferably said pattern comprises areas which are essentially free from SAPs, such as a clustered pattern. Such SAP-free zones are indicated in the cross sectional views of **figs. 1** **and** **2** as zones (30).

In a preferred embodiment, the nonwoven carrier, the bottom layer and/or the top layer, are hydrophilic. The nonwoven carrier, the bottom layer and/or the top layer can be naturally hydrophobic because they can be composed of polypropylene and/or polyester, respectively; the nonwoven carrier, the bottom layer and/or the top layer can be treated with a, preferably topical, hydrophilic treatment to increase absorption, such as a surfactant treatment.

In a second aspect, the invention provides an absorbent article comprising a nonwoven carrier according to an embodiment of the invention.

In a third aspect, the invention provides a process for manufacturing a nonwoven carrier according to an embodiment of the invention, comprising the steps of: providing a nonwoven bottom layer wherein the bottom layer is porous with a pore size smaller than that of the superabsorbent polymer particles and a top layer comprising staple and/or spunbond fibers, and binding, such as thermal binding, binding by melting by heat or pressure or ultrasonics, binding by needlepunching, chemical binding and/or binding by adhesives, preferably mechanically binding the staple and/or spunbond fibers to the nonwoven bottom layer thereby providing the nonwoven carrier. In a preferred embodiment, the bottom layer and the staple and/or spunbond fibers are bonded by hydroentanglement.

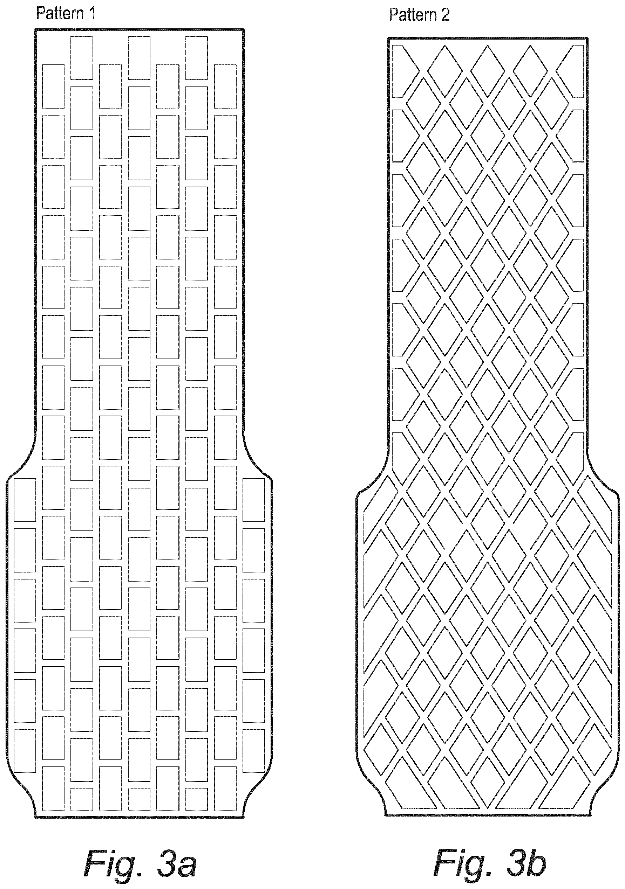

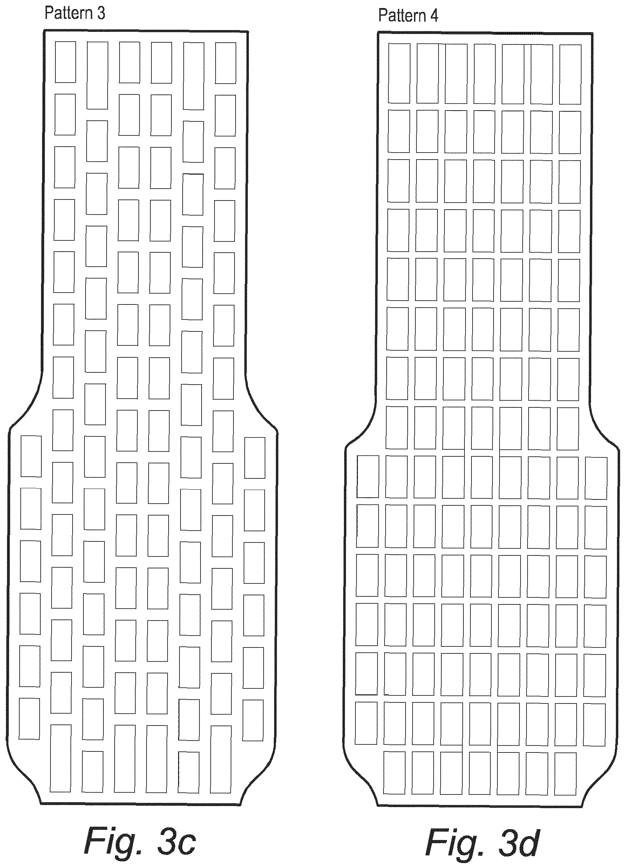

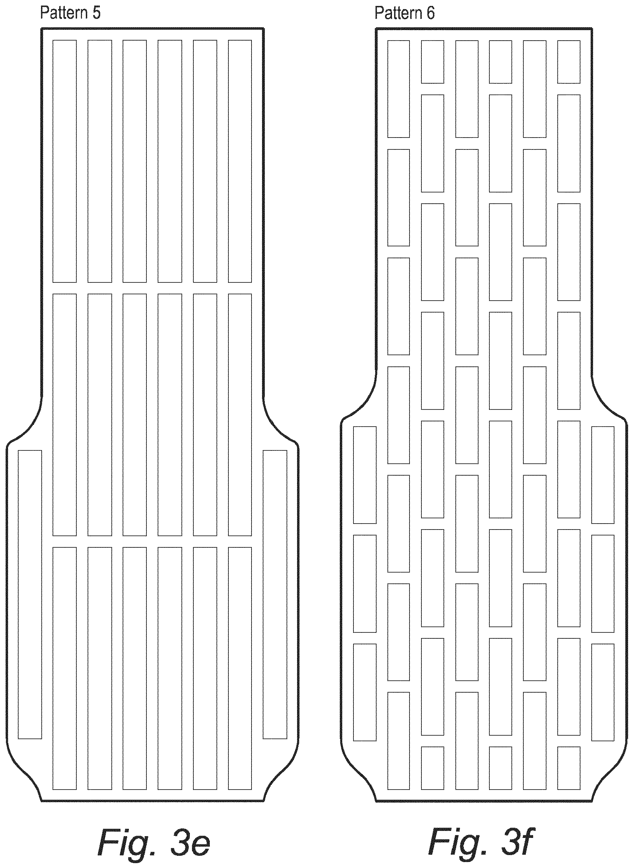

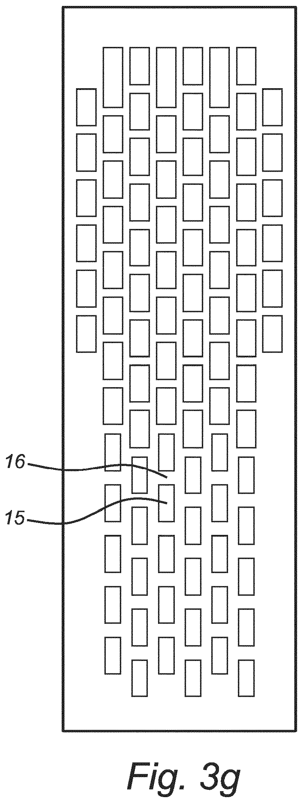

In a preferred embodiment, the process provides a way to distribute superabsorbent polymer particles over the carrier structure of the invention. Rather than only lying on the surface of the top layer, superabsorbent polymer particles will also be trapped inside the fibers. Their distribution is limited by the bottom layer. Its density prevents the particles from falling through the material. The superabsorbent polymer particles (28) are arranged in a pattern of discrete clusters (15) **(****Figs. 3a****-g).** In a preferred embodiment, those clusters (15) are rectangles, but other cluster shapes and patterns are possible as well. Other patterns are shown in **Figs. 3a****-g.** Along the SAP-free zones (16), the upper core wrap (4) is attached to the top-layer (7) of the nonwoven carrier (1) by thermoplastic adhesive (10). In dry state, the superabsorbent polymer particles (28) are contained in pocket-like compartments. In wet state, in order to allow full swelling of the superabsorbent polymer particles (28), those pocket-like compartments are no longer maintained. That is because of two effects: the adhesive joints between the staple fibers (19) and the upper core wrap (4) do partly break up, and the staple fibers (19) are relatively loosely bonded to the bottom layer (8), i.e. the free fiber length between bonding points is relatively long; as a result, even if the adhesive joints with the upper core wrap (4) stay intact, the staple fibers (19) do not exert a considerable force against the volume expansion of the absorbent core (18). Wet immobilization (i.e., immobilization of superabsorbent polymer particles when the disposable diaper and thus the nonwoven carrier at least partially is wetted) is improved due to entanglement between the top layer fibers (19) of the nonwoven carrier (1) and due to a part of the superabsorbent polymer particles (28) still sticking to the thermoplastic adhesive (10) on the upper core wrap (4).

In a preferred embodiment, the process further comprises the step of treating the nonwoven carrier (1), the bottom layer (8) and/or the top layer (7) with a surfactant after the hydroentanglement process. The nonwoven carrier (1), the bottom layer (8) and/or the top layer (7) are rendered hydrophilic through the use of a post treatment application of a surfactant. The hydrophilic characteristics of the nonwoven carrier (1), the bottom layer (8) and/or the top layer (7 enhance the wettability, and thus improve the absorbency when the carrier is employed for absorbent articles such as baby diapers or adult incontinence diapers.

In a preferred embodiment, the the top layer (7) is made of staple fibers which are provided in the form of a pre-made carded web, or the staple fibers are fed directly into the hydroentanglement process. Staple fibers are prefered for the top layer (7) because the carded web formation process is known to yield particularly bulky nonwoven webs. However, the top layer /7) can also be made of spun or spunmelt filaments that are bonded to the pre-made bottom layer (8) by any nonwoven bonding process known in the art, preferably by a hydroentanglement process. Alternatively, for the top layer (7) it is possible to employ bicomponent fibers where one of the components has a lower softening point than the other component, e.g. PE/PP, and include a heat transfer step, e.g. by hot air or an infrared lamp, in the process. In that case, the bottom layer (8) would also include similar bicomponent fibers, so that the top layer (7) and the bottom layer (8) are bonded as a result of the softening of the polymers with the lower softening point. The bicomponent fibers can have core/sheath or side/side or other configurations known in the art. It is preferable to use a side/side configuration because in that case the fibers will attain a crimped state by the heat transfer process. Instead of bicomponent staple fibers, the top layer (7) can also be made of bicomponent spun or spunmelt filaments.

In a preferred embodiment, the process further comprises the step of rolling up the nonwoven carrier.

In a further aspect, the invention provides a process for manufacturing an absorbent article according to an embodiment of the invention, comprising the steps of: providing a nonwoven carrier according to an embodiment of the invention and providing the top layer of the nonwoven carrier with superabsorbent polymer particles to manufacture an absorbent article.

In a final aspect, the invention provides for a use of a nonwoven carrier according to an embodiment of the invention for immobilizing superabsorbent polymer particles in an absorbent article, in particular in a diaper.

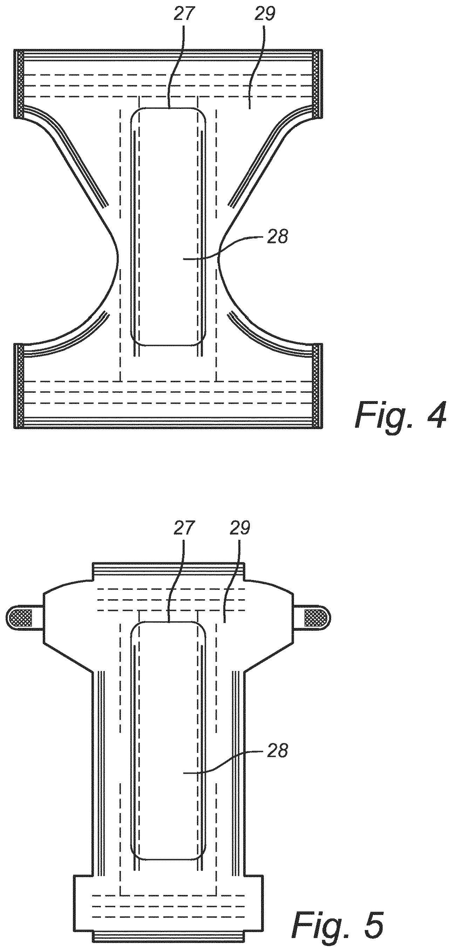

**FIGS. 4 and 5** are plan views of disposable diapers of a pant-type (adult) incontinence diaper and a baby diaper according to a certain embodiment of the present invention. The disposable diaper is shown in its flat out, uncontracted state (i.e. without elastic induced contraction). The central part of the disposable diaper (27) is shown which is limited by the outer part of the disposable diaper (29). The superabsorbent polymer particles (28) may be substantially continuously or clustered distributed within the central part of the disposable diaper (27).

Although the present invention has been described with reference to preferred embodiments thereof, many modifications and alternations may be made by a person having ordinary skill in the art without departing from the scope of this invention which is defined by the appended claims.

## Claims

1. A nonwoven carrier for immobilizing superabsorbent polymer particles in an absorbent article, comprising a nonwoven top layer, preferably comprising staple fibers and/or spunbond fibers, penetrable by the particles and a nonwoven bottom layer, whereby said top layer is bonded, wherein the bottom layer is porous with a pore size smaller than said particles.

2. The nonwoven carrier of claim 1, wherein the top layer comprises bicomponent fibers where one of the components has a lower softening point than the other component or the top layer is made of bicomponent spun or spunmelt filaments, preferably the bicomponent fibers having a core/sheath or side/side configu ration.

3. The nonwoven carrier of any of the preceding Claims, wherein the top layer is a carded nonwoven made of staple fibers or wherein the top layer consists of spun filaments.

4. The nonwoven carrier of any of the previous claims, whereby said top layer is mechanically bonded, thermally bonded, bonded by melting by heat or pressure or ultrasonics, bonded by needlepunching, chemically bonded and/or bonded by adhesives, preferably mechanically bonded, more preferably hydroentangled to the bottom layer.

5. The nonwoven carrier of any of the previous claims, wherein the bottom layer comprises bicomponent fibers where one of the components has a lower softening point than the other component, preferably the bicomponent fibers having a core/sheath or side/side configuration.

6. The nonwoven carrier of Claims 2 and/or 5, wherein the bicomponent fibers have a side/side configuration such that the fibers attain a crimped state by heat transfer.

7. The nonwoven carrier of any of the previous claims, wherein the grammage of the top layer (7) is between 1 to 200 gsm, preferably 5 to 100 gsm, more preferably 10 to 50 gsm, most preferably 15 to 40 gsm and the grammage of the bottom layer (8) is between 0,5 to 50 gsm, preferably 1 to 40 gsm, more preferably 2 to 30 gsm, more preferably 3 to 20 gsm, most preferably 4 to 12 gsm and/or whereby the staple fibers is a blend of at least two types of polyester, polyethylene and/or polypropylene fibers, preferably comprising at most 50% of a first type of fibers which are short and light, and preferably comprising at least 50% of a second type of long and heavy fibers.

8. The nonwoven carrier according to any of the previous claims, comprising superabsorbent polymer particles which are at least partially immobilized by the staple fibers and retained by the bottom layer.

9. The nonwoven carrier according to any of the previous claims, wherein the bottom layer has a pore size smaller than 100 µm.

10. The nonwoven carrier according to any of the previous claims, wherein the nonwoven carrier, the bottom layer and/or the top layer are hydrophilic.

11. Absorbent article comprising a nonwoven carrier according to any of claims 1 to 10.

12. Process for manufacturing a nonwoven carrier according to any of claims 1 to 10, comprising the steps of: providing a nonwoven bottom layer wherein the bottom layer is porous with a pore size smaller than that of the superabsorbent polymer particles and a top layer comprising staple fibers and/or spunbond fibers, and bonding the fibers to the nonwoven bottom layer thereby providing the nonwoven carrier, wherein the top layer and/or bottom layer comprises bicomponent fibers where one of the components has a lower softening point than the other component, and wherein the process comprises a heat transfer step applying heat preferably by hot air or an infrared lamp, wherein the bicomponent fibers have a side/side configuration such that the fibers attain a crimped state by heat transfer.

13. Process according to claim 12, wherein staple fibers are provided in the form of a pre-made carded web and/or the staple fibers are fed directly into a hydroentanglement process.

14. Process according to any of the claims 12 to 13, comprising the step of rolling up the nonwoven carrier.

15. Process for manufacturing an absorbent article according to claim 11, comprising the steps of:
- providing a nonwoven carrier according to the claims 1 to 10,
- providing the top layer of the nonwoven carrier with superabsorbent polymer particles to manufacture an absorbent article.

16. Use of a nonwoven carrier according to any of claims 1 to 10 for at least immobilizing superabsorbent polymer particles in a diaper.
